**Europäisches Patentamt**

**European Patent Office**

**Office européen des brevets**

(11) Veröffentlichungsnummer: **0 007 601**
B1

## (12) EUROPÄISCHE PATENTSCHRIFT

(45) Veröffentlichungstag der Patentschrift:
**14.04.82**

(21) Anmeldenummer: **79102582.8**

(22) Anmeldetag: **21.07.79**

(51) Int. Cl.³: **A 61 M 5/16,** C 08 L 25/04,
C 08 L 53/02

(54) Verwendung von Polystyrolpolymerisaten zur Herstellung von flexiblen Tropfkammern für Übertragungsgeräte.

(30) Priorität: **25.07.78 DE 2832504**

(43) Veröffentlichungstag der Anmeldung:
**06.02.80 Patentblatt 80/3**

(45) Bekanntmachung des Hinweises auf die Patenterteilung:
**14.04.82 Patentblatt 82/15**

(84) Benannte Vertragsstaaten:
**AT BE CH DE FR GB IT LU NL SE**

(56) Entgegenhaltungen:
**DE-A1-2 523 013**
**DE-A1-2 540 121**
**DE-A1-2 644 140**
**DE-B-2 116 073**
**US-A-3 021 841**
**US-A-3 233 457**
**US-A-3 316 908**

(73) Patentinhaber: **Intermedicat GmbH, Gerliswilstrasse 45, CH-6020 Emmenbrücke (CH)**

(72) Erfinder: **Bühler, Wolfgang, Dr., Ernstbergstrasse 8, Melsungen (DE)**

(74) Vertreter: **von Kreisler, Alek, Dipl.-Chem. et al, Deichmannhaus am Hauptbahnhof, D-5000 Köln 1 (DE)**

Verwendung von Polystyrol-Polymerisaten zur Herstellung von flexiblen Tropfkammern
für Übertragungsgeräte.

Die Erfindung betrifft die Verwendung von Polystyrol-Copolymerisate enthaltende Mischungen zur Herstellung transparenter, flexibler, mit energiereicher Strahlung in höheren Dosen sterilisierbarer Tropfkammerteile für Übertragungsgeräte.

Die zur Übertragung von Blut, Blutbestandteilen, Infusionslösungen und Plasmaexpandern aus einem Behälter in einen Empfänger üblicherweise verwendeten Übertragungsgeräte bestehen, einem allgemein üblichen und akzeptierten Konstruktionsschema entsprechend, aus dem zum Einstechen in den Flüssigkeitsbehälter als hohler Dorn ausgebildeten Einstechteil, einer Tropfkammer mit Tropfrohr zur Beurteilung der eingestellten Tropfenfolge, einem flexiblen Schlauch geeigneter Länge und einer zur Herstellung einer Leitungsverbindung, z.B. zu einem Katheter, dienenden Kegelverbindung. Eine in der Leitungsbahn angebrachte Flüssigkeitsregulierung oder durch äussere Einwirkungen erzielbare Querschnittsveränderungen dienen zur Kontrolle der Durchflussrate. Aus Kunststoff bestehende, zur einmaligen Verwendung vorgesehene Übertragungsgeräte der vorgenannten Art sind in DIN-Norm 58360 und 58362 standardisiert.

Die individuellen Ausführungsformen der Tropfkammer können sehr unterschiedlich sein. So sind Tropfkammern bekannt, die aus einem flexiblen transparenten Kunststoffrohr, z.B. einem Rohr aus weichgemachtem Polyvinylchlorid bestehen und durch Hochfrequenzschweissung bearbeitet werden und durch die Art des schlauchförmigen Ausgangsmaterials in einem Stück, also einteilig gefertigt sind. Eine andere Form wird aus zwei Formteilen zusammengesetzt, wobei das Formteil das Einstechteil und einen Teil der zylindrischen Kammer, das andere den übrigen Teil der Kammer und einen rohrförmigen Ansatz zur Befestigung des flexiblen Schlauchs umfasst.

Beide Teile können zur endgültigen Tropfkammer durch Verbindungsmethoden wie Kleben, Schweissen, formschlüssiges Ineinanderpassen luftdicht zusammengefügt werden.

Je nach Auswahl der verwendbaren Kunststoffe lässt sich eines der genannten Verbindungsverfahren anwenden, z.B. wird man für die Verbindung von Polyamidteilen oder Polystyrolteilen Klebeoder Schweissverfahren anwenden können, während das mechanische Zusammenfügen für die durch Kleben oder Schweissen nicht miteinander verbindbaren Kunststoffe vorzusehen ist. Eine viel verwendete Tropfkammer dieser letzten Art ist eine Kombination aus einem Polystyrol-Tropfkammeroberteil und einem Polypropylen-Tropfkammerunterteil.

Diese Materialanordnung hat indessen gravierende Nachteile, die nur durch konstruktive Hilfsmassnahmen überwunden werden können, um zu einem in der Anwendung sicheren und für einen Patienten, der eine Infusion oder Transfusion erhält, gefahrlosen Übertragungsgerät zu gelangen. Eine dieser Hilfsmassnahmen, um Tropfkammeroberteil und -unterteil flüssigkeits- und luftdicht im Gebrauch zusammenzuhalten, besteht darin, dass man die kreisrunde Verbindungsstelle der beiden Teile auf der Aussenseite mit einem Haltering umgibt, der z.B. durch das bekannte Spritzgiessverfahren auf der Tropfkammer geformt werden kann.

Durch entsprechende Gestaltung von Verankerungselementen, die mit dem angespritzten Ring in Wechselwirkung treten, wird eine ausreichend gute Dichtigkeit und mechanisch steife Stabilität der ineinander gesteckten Teile erreicht. Wird der den Haltering bildende Kunststoff so ausgewählt, dass er mit dem zugrunde liegenden Tropfkammermaterial verträglich, d.h. verschweissbar ist, wird durch eine beim Einspritzen der plastifizierten Kunststoffmasse auf die Tropfkammer entstehende Schweissverbindung eine ausgezeichnete Festigkeit zwischen den Tropfkammerteilen erreicht, während bei nicht verträglichen und daher nicht verschweissbaren Komponenten lediglich ein mechanischer Zusammenhalt resultiert.

Eine weitere Anforderung an moderne, aus zwei Teilen zusammengesetzte Tropfkammern besteht darin, dass deren Unterteile in gewissem Umfange flexibel sein müssen. Während einer Flüssigkeitsübertragung muss eine Tropfkammer zum Teil mit Flüssigkeit gefüllt sein, um als Luftfalle wirksam sein zu können. Die Füllung selbst wird in einfacher Weise dadurch bewerkstelligt, dass man auf die flexible Wand Druckbewegungen, z.B. durch Zusammendrücken mit den Fingern, einwirken lässt und durch diesen Pumpvorgang Luft aus der Tropfkammer herausdrückt und über der Tropfkammer im Infusionslösungsbehälter befindliche Flüssigkeit einströmen lässt.

Unter den üblicherweise zur Sterilisation von Kunststoffeinmalgeräten für die Medizin angewendeten Methoden wie Sterilisation mit Äthylenoxid oder Sterilisation mit energiereicher Strahlung ist letzterer Methode der Vorzug zu geben, da sie bei ausreichender Strahlendosis sowohl im Inneren lang gestreckter Konstruktionselemente, z.B. Schläuchen, Keimfreiheit besser gewährleistet als die Äthylenoxidsterilisation und weiterhin keine toxisch wirkenden Äthylenoxidgasrückstände oder daraus entstandene toxischen Folgeprodukte hinterlässt. Allerdings sind manche Kunststoffe, darunter z.B. das Polypropylen, bei den erforderlichen Strahlendosen von $3,5 \pm 10\%$ Mrad nicht mehr stabil und werden unter der Strahlungseinwirkung molekular abgebaut, was einer Versprödung des Materials entspricht. Zähigkeit und damit Funktionssicherheit sind unter diesen Behandlungsbedingungen nur noch bedingt gewährleistet.

Schliesslich ist es aus Gründen der Produktsicherheit erforderlich, dass der überwiegend aus weichgemachtem Polyvinylchlorid gefertigte Schlauch eines Übertragungsgerätes ausreichend zugfest mit dem Schlauchansatz der Tropfkammer verbunden ist. Mit Weich-PVC verklebbare Kunststoffe bereiten keine Schwierigkeiten, eine Zugfestigkeit von 20-30 N zu erreichen.

Es ist einzusehen, dass diese Werte bei schlecht verklebbaren Materialkombinationen wie Polyamid und Weich-PVC nicht erreicht werden und bei Polypropylen und Weich-PVC überhaupt nicht mehr möglich sind. Nach dem DE-U-Nr. 7213189 wird deshalb ein mit Lösemitteln oder Klebstofflösungen mit einem Weich-PVC-Schlauch kombinierbarer Schlauchansatz in einer Tropfkammer mit Polypropylen-Unterteil vorgeschlagen. Diese Verfahrensweise ist jedoch umständlich, material- und kostenaufwendig.

In der DE-B-Nr. 2116073 werden in enger Begrenzung thermoplastische Kautschuke auf Styrol-Butadien-Basis zur Verwendung in (Einmal)-Injektionsspritzen und für Pumpendichtungen beansprucht.

Diese handelsüblichen Materialien sind auch in dünneren Schichten von etwa 1 mm Dicke transluzent bis opak und in keinem Falle als optisch klare Werkstoffe anzusprechen. Auch bekannte Polymermischungen der thermoplastischen Styrol-Butadien-Kautschuke mit z.B. Polyolefinen wie Polyäthylen und Polypropylen sind praktisch lichtundurchlässig und erscheinen daher wie mit Farbmitteln deckend eingefärbte Kunststoffe. Es ist auch bekannt, dass die bislang üblichen schlagfesten Polystyrole (impact modified polystyrene) z.B. durch eine Pfropfungspolymerisation aus Styrol und Butadien(-Latex) hergestellt werden und wegen der unterschiedlichen, im optischen Verhalten nicht kompatiblen Zustandsphasen beider Polymere nicht transparent sind, sondern ein milchiges Aussehen haben.

Es lag daher nicht nahe, sowohl für ein ganz anderes Anwendungsgebiet, nämlich die Herstellung von flexiblen und transparenten Formteilen für sogenannte Übertragungsgeräte und hier speziell die sogenannten Tropfkammern, als auch im Hinblick auf den Stand der Technik der nichttransparenten Butadien-modifizierten Polystyrol-Typen (schlagfeste Polystyrole) eine Mischung mit bislang im Rahmen der technischen Möglichkeiten allein zugänglichen steifen und ein hohes E-Modul aufweisenden Polystyrol-Homo- oder Copolymerisaten (letztere z.B. SAN, Styrol-Acrylnitril-Polymerisate) mit thermoplastischen SB-(SBS) oder SIS-Kautschuken herzustellen, die die für den Anwendungsfall unbedingt wichtigen Eigenschaften der Transparenz, der Flexibilität und dabei ausreichender Zähigkeit aufweist.

Durch die theoretische Kombination von Eigenschaftsbildern der ersten und der zweiten Komponente wird nicht nahegelegt, dass sich in der Summierung aller genannten Eigenschaften ein für den vorgesehenen Zweck auch brauchbares Material ergeben könnte.

Gerade die in der DE-A1-Nr. 2644140 genannten Materialien sind ausdrücklich als starre Werkstoffe gekennzeichnet, wie in Anspruch 10 ausgeführt ist, und auf S. 8, Abs. 1, wird ein starres Material gefordert, wie es in besonderem Masse die genannten Polymeren Polystyrol und Polymethacrylat sind. Die Notwendigkeit bzw. Zweckmässigkeit eines flexiblen Schaubehälters wird hier ausdrücklich verneint und seine Verwendung nicht angestrebt.

Eine gleichartige Angabe ist in der US-A Nr. 3233457, Spalte 3, Zeilen 31 und 32, zu finden, wo ebenfalls (starre) Acryl-Polymeren, Polystyrol oder Vinyl(chlorid)-Polymere genannt sind. Wenn in den vorangehenden Zeilen 27 bis 31 flexible oder starre Kunststoffe genannt sind, muss auch für die im folgenden genannten 3 Werkstoffe eine Zuordnung getroffen werden: Es ist dem Fachmann bekannt, dass unter *acrylic(s)* Polymethylacrylate, Polymethylmetacrylate and andere Acrylsäureester-Polymere zu verstehen sind, und dass diese ohne Zweifel starre Werkstoffe darstellen. Dasselbe gilt für *polystyrene*, das zum Zeitpunkt dieser Anmeldung (1962) nur als starres Material verfügbar war und erst durch die vorliegende Erfindung als flexible Modifikation in transparenter Einstellung zugänglich gemacht wird. *Vinyl plastics* können dem Sprachgebrauch nach sowohl weichmacherhaltige (=flexibel) als auch weichmacherfreie (=starre) Vinyl(chlorid)-Polymere sein.

Aus diesem Text ist keine Lehre herzuleiten, dass ein flexibles, transparentes Merkmal dem Begriff *polystyrene* zugeordnet werden sollte und demnach als bekannt vorauszusetzen wäre.

In der US-A Nr. 3316908 wird Acrylnitril-Butadien-Styrol als Styrol-Copolymerisat genannt.

Daraus ergibt sich die Aufgabe, für die aus Ober- und Unterteil bestehende Tropfkammer nach Funktions- und Herstellungskriterien ein Material zu verwenden, mit dem folgende Anforderungen optimal erfüllt werden:

1. Transparenz zur Kontrolle der Tropfkammerfüllung und Beurteilung der Tropfenfolge als Mass der Durchflussrate.

2. Flexibilität und Rückstellvermögen des Tropfkammerunterteils zur teilweisen Füllung des Unterteils durch Zusammendrücken.

3. Beständigkeit gegen energiereiche Strahlung im Bereich ab 3,5 Mrad.

4. Integrierte Anordnung von Tropfkammerunterteil und Schlauchansatz zum Ankleben eines üblichen Weich-PVC-Schlauchs mit Lösemitteln oder Klebstofflösungen.

5. Im Hinblick auf Verschweissbarkeit mit einander verträglicher Kunststoffmaterialien für Tropfkammeroberteil und -unterteil, um entweder eine flüssigkeits- und luftdichte Verbindung mittels bekannter Fügeverfahren wie Ultraschallschweissungen, Wärmeimpulsschweissen, Reibschweissen, Spiegelschweissungen usw. oder durch Umspritzen der zusammengesetzten Tropfkammerteile mit einem im gleichen Sinne kompatiblen Kunststoff zu erreichen.

6. Integrierte Anordnung von Tropfkammeroberteil und Einstechteil, das zum Perforieren von Verschlüssen von Infusionslösungsbehältern geeignet sein, und daher ausreichende Härte und Steifigkeit aufweisen muss.

Die Erfindung betrifft die Verwendung von Polystyrol-Polymerisaten zur Herstellung von Tropfkammern für Übertragungsgeräte, wobei das Oberteil der Tropfkammer aus Polystyrol-Homo- oder Copolymerisaten besteht, und ist dadurch gekennzeichnet, dass zur Herstellung des flexiblen Unterteils der Tropfkammer ein Gemisch oder eine Legierung von

a) Polystyrol-Homo- oder Copolymerisaten mit
b) thermoplastischen Kautschuken auf der Basis von Styrol-Butadien-Styrol oder Styrol-Isopren-Styrol-Blockpolymerisaten verwendet werden.

Dem obigen Anforderungsprofil entsprechend wird vorgeschlagen, teilweise flexible Tropfkammern aus Polystyrol herzustellen, und zwar das Tropfkammeroberteil aus einem Polystyrol-Homo- oder Copolymerisat mit hoher Steifigkeit und Härte, das den Anforderungen nach 1., 3., 5. und 6. entspricht. Geeignete Polymerisate sind Normalpolystyrol (Homopolymerisate), Copolymerisate auf Basis Styrol-Acrylnitril (SAN), Acrylnitril-Butadien-Styrol (ABS), schlagzäh modifiziertes Polystyrol auf Basis Styrol-Butadien (SB), Methylmethacrylat-Butadien-Styrol (MBS) und Methylacrylat-Styrol-Acrylnitril (ASA) und die dem Fachmann bekannten Modifikationen dieser Systeme, und das flexible Tropfkammerunterteil aus einem Blend aus 1. Polystyrol-Homo- oder -Copolymerisat und 2. thermoplastischem Kautschuk auf Basis Styrol-Butadien-Styrol (SBS) oder Styrol-Isopren-Styrol (SIS) Blockcopolymerisaten, das den Anforderungen 1 bis 6 entspricht.

Zur Herstellung des Blends geeignete Polymerisate nach 1. sind Copolymerisate auf Basis Styrol-Butadien (SB) und Methacrylat-Butadien-Styrol (MBS). Prinzipiell eignen sich hierzu die mit thermoplastischen Kautschuken auf Styrol-Butadien-Styrol- oder Styrol-Isopren-Styrol-Basis mischbaren Polystyrol-Homo- und Copolymerisate, deren Brechungsindices $n^{20}$ so kompatibel sind, dass weitgehend transparente Mischungen mit hohem Transmissionsgrad im sichtbaren Licht resultieren.

Darüberhinaus lässt sich das vorgeschlagene Prinzip zur Blendherstellung auf andere Polymergruppen ausser Styrol-Polymerisaten anwenden, da Styrol-Butadien-Styrol bzw. Styrol-Isopren-Styrol-Blockcopolymerisate mit einer weiteren Anzahl von Polymeren, z.B. der Gruppe der Polyolefine (Polyäthylen, Äthylen-Vinylacetat-Copolymere, Polypropylen, Polybuten, Polymethylpenten) oder der Polyacrylsäureestergruppe mischbar sind.

Neuartige Styrol-Butadiencopolymerisate zeichnen sich ebenso wie die seit langem bekannten Polystyrolhomopolymerisate und einige Styrolcopolymerisate auf Basis Styrol-Acrylnitril oder Methacrylsäureester-Butadien-Styrol durch weitgehende Transparenz aus und bieten darüberhinaus wesentlich verbesserte Zähigkeit und Schlagzähigkeit, weshalb sie zur Herstellung flexibler und gegen mechanische Beanspruchung erhöht widerstandsfähiger Spritzgussteile mit Vorteil eingesetzt werden können. Trotz der im Vergleich mit konventionellen Styrolhomopolymerisaten höheren Flexibilität reicht diese in manchen Anwendungsfällen nicht aus, um die angestrebte Funktion einer flexiblen Tropfkammer zu gewährleisten und es wird deshalb vorgeschlagen, thermoplastische Kautschuke auf Basis Styrol-Butadien-Styrol oder Styrol-Isopren-Styrol für eine ausreichende Flexibilität bei gleichbleibender Transparenz einzumischen.

Thermoplastische Kautschuke sind Blockcopolymere mit der schematisierten Sequenz SSSSS BBBBBBBBBB SSSSS (S=Styrolmolekül, B=Butadienmolekül). Besonders geeignet sind Dreiblockcopolymere oder Radialblockcopolymere des S-B-S-Typs, bei denen die äusseren Blöcke S der Polymerkette identische Polystyrolsegmente mit glasartigem, thermoplastischen Charakter sind. Der kautschukartige Mittelblock B ist elastisch und besteht aus Polybutadien oder Polyisopren. Die Polystyrolphase ist bei einem bestimmten prozentualen Anteil getrennt und besteht aus kleinen Aggregaten, die in der kontinuierlichen Polybutadien- oder Polyisoprenmatrix eingebettet und so klein sind, dass es zu keiner Streuung des sichtbaren Lichts kommt, was eine Transparenz des Polymeren bedeutet.

Es wurde gefunden, dass sich Mischungen (Blends) aus Styrolcopolymerisaten und thermoplastischem Kautschuk auf Basis Styrol-Butadien-Styrol oder Styrol-Isopren-Styrol hervorragend zur Herstellung von flexiblen, transparenten, verklebbaren und verschweissbaren Tropfkammerunterteilen verwenden lassen, wenn die beiden Komponenten in geeigneten Mischungsverhältnissen gemischt, homogenisiert und im Spritzgiessverfahren zu Formteilen verarbeitet werden.

Vorzugsweise werden als Styrolcopolymerisate bzw. diesen entsprechende Polymermischungen schlagfeste, transparente Styrol-Butadien-Copolymerisate oder Methacrylat-Butadien-Styrol-Polymermischungen eingesetzt.

Geeignete Formteile werden erhalten, wenn aus 30-90% eines Polystyrol-Copolymerisats bzw. einer entsprechenden Polymermischung, vorzugsweise 60-80% mit 70-10%, vorzugsweise 40-20%, eines thermoplastischen Kautschuks auf Styrol-Butadien-(Isopren)-Styrolbasis ein Compound hergestellt und im Spritzgussverfahren verarbeitet wird.

Beispiele für geeignete Blends sind im folgenden angegeben:

*Beispiel 1*

60 Gewichtsprozente eines Polymergemisches auf Methacrylat-Butadien-Styrol-Basis (MBS) in Granulatform werden mit 40 Gewichtsprozenten eines partikelförmigen oder granulierten thermoplastischen Styrol-Butadien-Styrol-Kautschuks in einer Mischvorrichtung, z.B. Taumelmischer, gut vermischt und in einem Mischextruder oder Innenmischer homogen aufgeschmolzen, extrudiert und granuliert. Das erhaltene Granulat wird im Spritzgiessverfahren zu einem Tropfkammerunterteil geformt und das so erhaltene Teil mit einem Tropfkammeroberteil durch Ultraschallschweissung verbunden. Die in einem Übertragungsgerät für Infusionslösungen inkorporierte

Tropfkammer ist voll funktionsfähig und erfüllt die Anforderungen an Dichtigkeit, Transparenz, Flexibilität und Materialbeständigkeit bei Äthylenoxid- und Gammasterilisation.

*Beispiel 2*

75 Gewichtsprozente eines transparenten Polystyrol-Butadien-Copolymerisats in Granulatform werden mit 25 Gewichtsprozenten eines partikelförmigen oder granulierten thermoplastischen Styrol-Butadien-Styrol-Kautschuks in einer Mischvorrichtung gemischt und in einem Mischextruder homogen aufgeschmolzen, extrudiert und granuliert. Das so erhaltene Granulat ist für die Spritzgiessverarbeitung geeignet und es werden mit einem entsprechenden Spritzgiesswerkzeug daraus Tropfkammerunterteile gefertigt. Mit einem Tropfkammeroberteil kombiniert, werden beide Teile miteinander verbunden. Die erhaltene Tropfkammer als Teil eines Übertragungsgeräts entspricht vollständig den an ein derartiges Teil zu stellenden Anforderungen wie Dichtigkeit, Transparenz, Flexibilität, Verklebbarkeit und Beständigkeit bei einer Sterilisation mit energiereicher Strahlung oder mit Äthylenoxid.

**Patentansprüche**

1. Verwendung von Polystyrolpolymerisaten zur Herstellung von Tropfkammern für Übertragungsgeräte, wobei das Oberteil der Tropfkammer aus Polystyrol-Homo- oder Copolymerisaten besteht, dadurch gekennzeichnet, dass zur Herstellung des flexiblen Unterteils der Tropfkammer ein Gemisch oder eine Legierung von
a) Polystyrol-Homo- oder -Copolymerisaten mit
b) thermoplastischen Kautschuken auf der Basis von Styrol-Butadien-Styrol oder Styrol-Isopren-Styrol-Blockpolymerisaten verwendet werden.
2. Verwendung nach Anspruch 1, dadurch gekennzeichnet, dass die Polystyrol-Copolymerisate Copolymerisate auf Basis Styrol-Butadien und Methacrylat-Butadien-Styrol sind.

**Claims**

1. Use of polystyrene polymers for the production of drip chambers for transfusion apparatus, the upper part of the drip chamber consisting of polystyrene homopolymers or copolymers, characterized in that a mixture or alloy of
a) polystyrene homopolymers or copolymers, with
b) thermoplastic rubbers based on styrene-butadiene-styrene or styrene-isoprene-styrene block polymers are used for producing the flexible lower part of the drip chamber.
2. Use according to claim 1, characterized in that the polystyrene copolymers are copolymers based on styrene-butadiene and methacrylate-butadiene-styrene.

**Revendications**

1. Utilisation de polymérisats de polystyrène pour la réalisation de chambres de goutte-à-goutte pour appareils de transfert, dans lesquels la partie supérieure de la chambre de goutte-à-goutte est composée d'homo- ou de copolymérisats de polystyrène, caractérisée en ce que l'on utilise pour la réalisation de la partie inférieure souple de la chambre de goutte-à-goutte un mélange ou un alliage
a) d'homo- ou copolymérisats de polystyrène, avec
b) des caoutchoucs thermoplastiques à base de polymérisats séquencés de styrène-butadiène-styrène ou de styrène-isoprène-styrène.
2. Utilisation selon la revendication 1, caractérisée en ce que les copolymérisats de polystyrène sont des copolymérisats à base de styrène-butadiène et de méthacrylate-butadiène-styrène.